# EUROPEAN PATENT SPECIFICATION

(11) **EP 2 018 832 B1**
(45) Date of publication and mention of the grant of the patent: **21.09.2011**
(21) Application number: 07014709.5
(22) Date of filing: 26.07.2007
(51) Int. Cl.: A61F 2/44

(54) **Intervertebral disc prosthesis**
Bandscheibenprothese
Prothèse de disque intervertébral

(43) Date of publication of application: 28.01.2009
(73) Proprietor: BIEDERMANN MOTECH GmbH, 78054 Villingen-Schwenningen (DE)
(72) Inventor: Biedermann, Lutz, 78048 VS-Villingen (DE); Matthis, Wilfried, 79367 Weisweil (DE); Harms, Jürgen, 76227 Karlsruhe (DE)
(74) Representative: Hofer, Dorothea

(56) References cited:
- WO-A-99/65425
- WO-A-2004/037131
- WO-A-2005/009299
- DE-A1- 10 130 825

## Description

The invention relates to an intervertebral disc prosthesis according to the features of claim 1.

An intervertebral disc prosthesis using metal springs arranged between a base plate and a top plate is known from, for example, US 4,309,777. This artificial intervertebral disc comprises an upper and a lower disc portion with a plurality of helical springs extending from the upper to the lower disc portion which yieldably urge the disc portions away from each other.

US 6,770,094 B2 describes an intervertebral disc prosthesis comprising a cranial disc and a caudal disc, which are supported against each other elastically upon compression by springs. The springs consist of a memory-metal alloy which exhibits super-elastic properties at body temperature. In one embodiment the springs are formed by leaf springs which have the form of strips, one end of which is mounted on the upper disc and the other end of which is mounted on the lower disc.

US 7,201,776 B2 discloses an artificial disc replacement (ADR) including a pair of opposing endplate components, each attached to one of the upper and lower vertebrae, a cushioning component disposed between the endplate components; and a filler material contained within the cushioning component. In one embodiment the cushioning component is a tire-like component which cooperates with a metal hub in the center of the device which holds the air, fluid, gel or other material within the tire.

Commercially available intervertebral disc prostheses may have one ore several disadvantages such as no axial damping, no rotational control, no restoring in all degrees of freedom and a Range of Motion (ROM) which is excessive if compared to a natural disc.

WO 2004/037131 A1 describes an intervertebral disc prosthesis according to the preamble of claim 1.

It is the object of the invention to provide an intervertebral disc prosthesis which has improved axial damping combined with rotational control and flexion/extension control.

The object is solved by an intervertebral disc prosthesis according to claim 1. Further developments are given in the dependent claims.

The intervertebral disc prosthesis according to the invention achieves high axial damping by means of the loop-shaped springs with the loop portion directed away from the central axis. Since the springs are connected with their free ends to the base plate and the top plate, respectively, a rotational control is achieved. The Range of Motion (ROM) in flexion/extension is reduced to approximately +/- 5° which protects the surrounding joints, in particular the facet joints.

The end stop preferably made of an elastomer material additionally restricts and damps the axial movement.

The flexible protection sleeves prevents vessels and tissue from growing into the space between the springs.

Further features and advantages of the invention will become apparent to those skilled in the art from the following description of the preferred embodiments of the invention which have been shown and described by way of illustration. As will be realized, the invention is capable of other and different embodiments, and its details are capable of modification in various respects. Accordingly, the drawings and the description are to be regarded as illustrative in nature and not as restrictive.

Fig. 1 shows as sectional view of the intervertebral disc prosthesis according to an embodiment of the invention.

Fig. 2 shows a perspective side view, partially in section of the intervertebral disc prosthesis according to Fig. 1

Fig. 3 shows a perspective exploded view of the intervertebral disc prosthesis.

Fig. 4 shows a perspective side view of the intervertebral disc prosthesis.

Fig. 5 shows a portion of the intervertebral disc which is used for clamping the springs to the top plate.

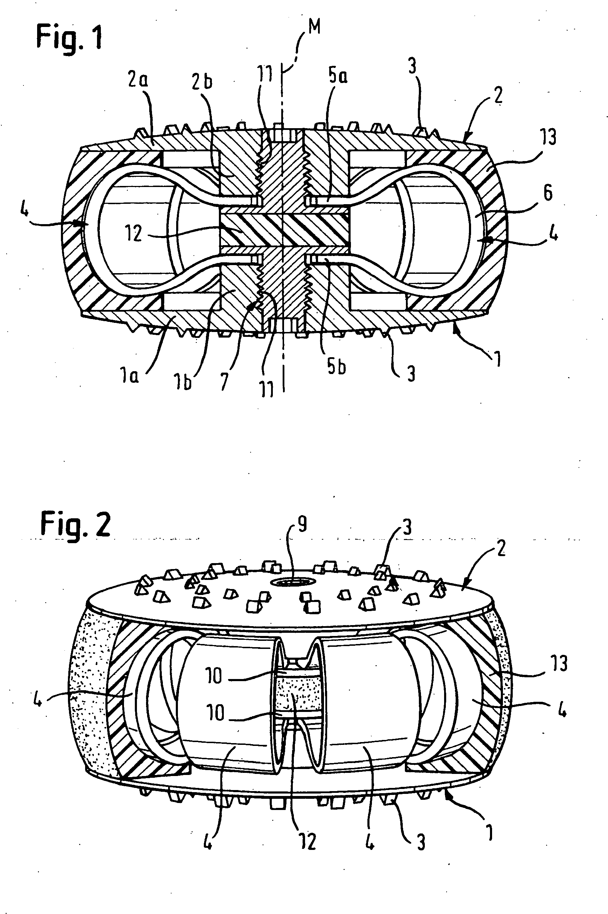

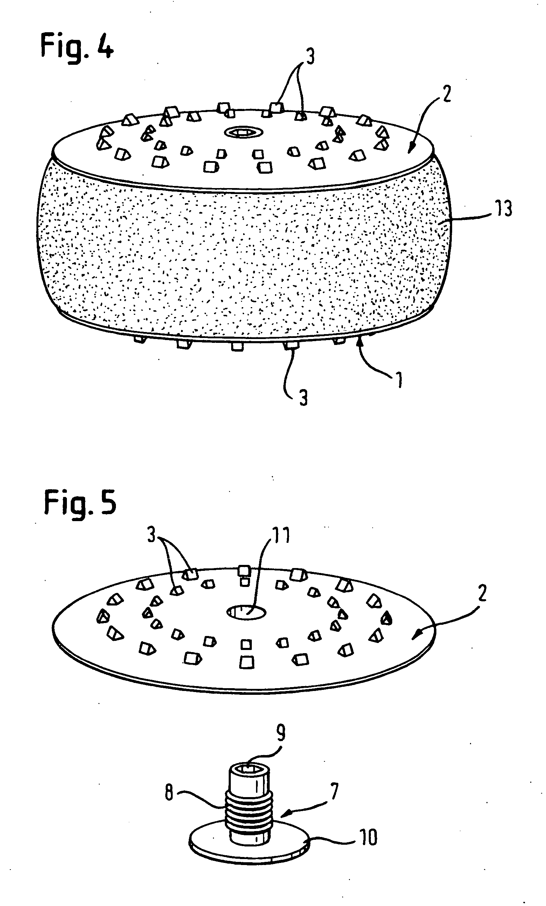

As can be seen from Figures 1 to 4. the intervertebral disc prostheses comprises a base plate 1 and a top plate 2 opposite to the base plate. The base plate 1 and the top plate 2 each have a plate portion 1a, 2a and a central cylindrical projection 1b,2b. The base plate 1 and the top plate 2 are arranged such that the cylindrical projection 1b, 2b are facing each other. A central axis M extends through the center of the projections 1b,2b. A threaded bore 11 extends through each of the base plate 1 and the top plate 2 which is coaxial with the cylindrical projection 1b, 2b. The plate portions 1a, 2a have in the embodiment shown a circular shape but can have any other shape, in particular a shape adapted to the contour of the end plates of the adjacent vertebrae. The plate portions 1a, 2a are slightly curved outward and each plate portion comprises a plurality of teeth 3 for anchoring in the end plates of the adjacent vertebrae.

The base plate 1 and the top plate 2 are made of biocompatible material, such as titanium or stainless steel or a biocompatible plastic material such as for example polyaryletheretherketone (PEEK).

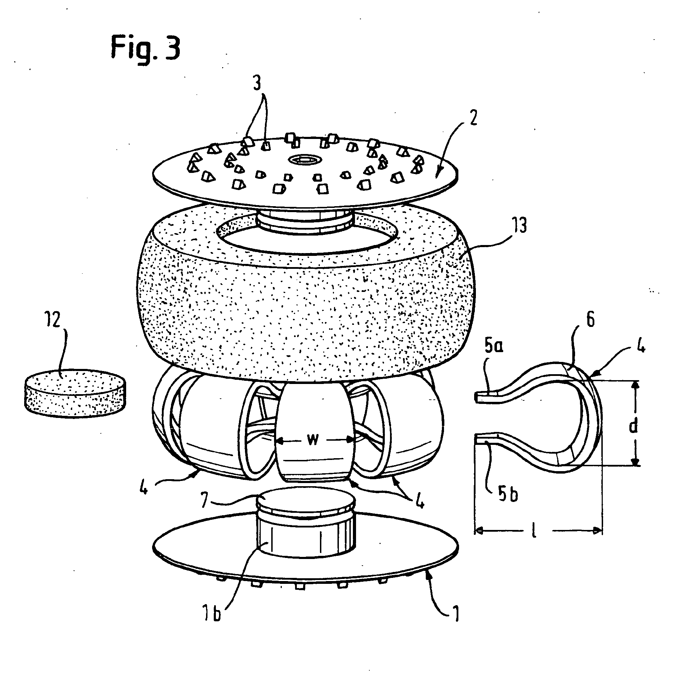

A plurality of springs 4 are arranged between the base plate 1 and the top plate 2. As can be seen in particular in Fig. 3 each spring 4 has the shape of a substantially flat strip similar to a leaf spring and comprises two free end sections 5a,5b and a loop-shaped bent section 6 between the free end sections 5a,5b. In a preferred embodiment the springs are leaf springs.

The end sections 5a,5b are straight flat sections as can be seen in particular in Fig. 1. The width of the strip which forms the spring 4 is such that a plurality, for example, six springs can be arranged around the central axis M with a space between adjacent springs. The width w is largest at the outermost part of the loop-shaped section 6 and decreases towards the end sections 5a,5b as can be seen in Fig. 3. The length 1 of the spring 4 from the end sections 5a,5b up to the outermost part of the loop-shaped section 6 is such that when the spring is mounted the loop-shaped section 6 lies completely between the plate portions 1a,2a. The largest diameter d of the loop-shaped section 6 in a direction parallel to the central axis M of the prosthesis is such that in the mounted state shown in Fig. 1 and 2 there is a distance between the spring 4 and the base plate 1 and the top plate 2.

The springs 4 can be made of any highly flexible biocompatible material. For example, the spring can be made of a shape-memory material exhibiting super-elasticity, in particular of a shape-memory metal such as a nickel titanium alloy. A specific example for this alloy is nitinol.

As can be seen in Figures 1, 3 and 5 mounting screws 7 are provided for mounting the springs between the base plate 1 and the top plate 2. Each mounting screw 7 comprises a threaded screw section 8 and recess 9 at the free end thereof for engagement with a screwing-in tool. Opposite to the free end the mounting screw 7 comprises an disc-shaped portion 10 the diameter of which corresponds to the diameter of the cylindrical projection 1b,2b. Each screw section 8 engages with the coaxial threaded bore 11 extending through the base plate 1 and its cylindrical projection 1b and through the top plate 2 and its cylindrical projection 2b, respectively. The length of the screw section is such that when the mounting screws 7 are connected to the base plate 1 and the top plate 2 respectively and the spring 4 is clamped by the disc-shaped portion 10 and the cylindrical projection 1b, 2b, the recess 9 can be engaged with the screwing-in tool. The mounting screws 7 are made of a biocompatible material like the base plate and the top plate.

Between the disc-shaped portions 10 of the mounting screws 7 a damping element 12 is provided. Preferably the damping element is shaped as a disc and more preferably is made of an elastomer material such as polycarbonate uretane (PCU). The thickness of the disc is selected such that it fills the space between the mounting screws 7. The material is selected such that the desired damping effect can be achieved.

As can be seen in Figures 1 to 4 a protective sleeve 13 is provided which surrounds the springs 4 circumferentially and extends from the base plate 1 to the top plate 2. In a radial direction, the sleeve 13 partially encompasses the loop-shaped sections 6 of the springs 4. Hence, the loop-shaped sections 6 are not in direct contact with the plate portions 1a, 2a of the base plate 1 and the top plate 2. This leads to a further axial damping. The protective sleeve 13 is made of a flexible material, in particular it can be made of an elastomer material such as polycarbonate uretane (PCU).

The intervertebral disc prosthesis is pre-assembled in such a way that the springs 4 are clamped between the disc-shaped portions 10 of the mounting screws 7 and the cylindrical projections 1b, 2b of the base plate 1 and the top plate 2, respectively. The damping element 12 may be fixed to the free side of the disc-shaped portion 10 of the mounting screw, for example, by glueing. When all the springs are clamped the protective sleeve is mounted.

In use, the intervertebral disc prosthesis is inserted between two adjacent vertrebrae to replace an intervertebral disc. The teeth 3 on the outer surface of the base plate and the top plate engage into the end plates of the adjacent vertebrae. During flexion and extension of the spine pressure is exerted onto or is relieved from one or several of the springs so that they are compressed or extended. Simultaneously the damping element 12 damps the purely axial motion. The springs 4 also provide rotational control, since the end sections 5a,5b are fixed which causes the loop-shaped sections 6 to be twisted to a certain degree when a torsional motion of the base plate and the top plate against each other occurs.

The Elasticity of the springs and the shape of the springs limits the Range of Motion (ROM) in flexion/extension to approximately +/- 5° to protect neighbouring structures such as facet joints and ligaments against overloading. The substantial axial dampening, which is similar to that of a natural disc, limits axial compression forces to the physiological range and allows elastic interaction of the artificial disc and the facet joints ("lock facets").

Modifications are possible. The shape of the base plate and the top plate can be adapted to more anatomically fit to the end plates of the vertebrae. Hence, it can be kidney-shaped or oval-shaped or otherwise shaped. The teeth can have another shape as that shown. For example, the teeth can be spikes or can have asymmetric shape. Furthermore, the outer surface of the base plate 1 and the top plate 2 can be roughened or coated for improving growth-in into the end plates.

The springs can be mounted by screwing it directly to the projection 1b,2b of the base plate 1 or the top plate 2 instead of clamping them.

The damping element 12 can be realized otherwise, for example by a hydrogel cushion or by other kinds of springs, such as a helical spring or disc springs.

The damping element 12 can also be omitted, although in this case the advantage of the axial damping is reduced. In addition, the protective sleeve can be omitted.

## Claims

1. Intervertebral disc prosthesis comprising
a base plate (1),
a top plate (2), a central axis (M) extending through the center of the base plate and the top plate,
at least two springs (4) arranged between the base plate and the top plate, the springs each having a loop-shaped section (6) and two free ends (5a, 5b), wherein
one of the free ends (5a) of each spring is connected to the base plate and the other one of the free ends is connected to the top plate, **characterized in that** the loop-shaped section is directed away from the central axis, and
that a plurality of loop-shaped springs (4) are provided in a circular arrangement around the central axis (M) wherein the springs are adjacent to each other in a circumferential direction of said circular arrangement with a space between the adjacent springs (4).

2. Intervertebral disc prosthesis according to claim 1, wherein the spring (4) is a substantially flat strip.

3. Intervertebral disc prosthesis according to claim 1 or 2, wherein a clamping device (7) is provided which clamps the free end sections (5a, 5b) to the base plate (1) and the top plate (2)

4. Intervertebral disc prosthesis according to one of claims 1 to 3, wherein the base plate (1) and the top plate (2) each have a projection (1b, 2b) coaxial with the central axis, the projections facing each other.

5. Intervertebral disc prosthesis according to claim 4, wherein the end sections (5a, 5b) of the springs are clamped to the projections (1b, 2b).

6. Intervertebral disc prosthesis according to one of claims 1 to 5, wherein a damping element (12) is arranged between the base plate and the top plate, the damping element being preferably an elastomer.

7. Intervertebral disc prosthesis according to one of claims 1 to 6, wherein the springs (4) are made from a material exhibiting super-elasticity, preferably from a shape-memory alloy such as nickel titanium.

8. Intervertebral disc prosthesis according to one of claims 1 to 7, wherein a flexible protection sleeve (13) is arranged between the base plate and the top plate.

9. Intervertebral disc prosthesis according to one of claims 1 to 8, wherein the sides of the base plate and the top plate which are opposite to the springs (4) comprise an engagement structure (3) for engagement with an adjacent end plate of a vertebra.

10. Intervertebral disc prosthesis according to one of claims 1 to 9, wherein the free ends are adjacent the loop-shaped section.

## Patentansprüche

1. Bandscheibenprothese mit
einer Basisplatte (1),
einer oberen Platte (2), einer Mittelachse (M), die sich durch die Mitte der Basisplatte und der oberen Platte erstreckt,
zumindest zwei Federn (4), die zwischen der Basisplatte und der oberen Platte angeordnet sind, wobei die Federn jeweils einen schleifenförmigen Bereich (6) und zwei freie Enden (5a, 5b) haben, wobei
eines der freien Enden (5a) der jeweiligen Feder mit der Basisplatte verbunden ist, und das andere der freien Enden mit der oberen Platte verbunden ist,
**dadurch gekennzeichnet, dass**
der schleifenförmige Bereich von der Mittelachse weggerichtet ist, und
dass viele schleifenförmige Federn (4) in einer runden Anordnung um die Mittelachse (M) vorgesehen sind, wobei die Federn in einer Umfangsrichtung der runden Anordnung aneinander angrenzen und zwischen den angrenzenden Federn (4) ein Raum ist.

2. Bandscheibenprothese gemäß Anspruch 1,
wobei die Feder (4) ein im Wesentlichen flacher Streifen ist.

3. Bandscheibenprothese gemäß Anspruch 1 oder 2,
wobei eine Klemmvorrichtung (7) vorgesehen ist, die die freien Endbereiche (5a, 5b) an die Basisplatte (1) und die obere Platte (2) klemmt.

4. Bandscheibenprothese gemäß einem der Ansprüche 1 bis 3, wobei die Basisplatte (1) und die obere Platte (2) jeweils einen Vorsprung (1b, 2b) haben, der koaxial zu der Mittelachse ist, wobei sich die Vorsprünge einander gegenüber liegen.

5. Bandscheibenprothese gemäß Anspruch 4,
wobei die Endbereiche (5a, 5b) der Federn an die Vorsprünge (1b, 2b) geklemmt sind.

6. Bandscheibenprothese gemäß einem der Ansprüche 1 bis 5, wobei ein Dämpfelement (12) zwischen der Basisplatte und der oberen Platte angeordnet ist, wobei das Dämpfelement vorzugsweise ein Elastomer ist.

7. Bandscheibenprothese gemäß einem der Ansprüche 1 bis 6, wobei die Federn (4) aus einem Material geschaffen sind, das eine Superelastizität zeigt, vorzugsweise aus einer Formgedächtnislegierung wie zum Beispiel Nickeltitan.

8. Bandscheibenprothese gemäß einem der Ansprüche 1 bis 7, wobei eine flexible Schutzbuchse (13) zwischen der Basisplatte und der oberen Platte angeordnet ist.

9. Bandscheibenprothese gemäß einem der Ansprüche 1 bis 8, wobei die Seiten der Basisplatte und der oberen Platte, die den Federn (4) zugewandt sind, eine Eingriffsstruktur (3) für einen Eingriff mit einer angrenzenden Endplatte eines Wirbels aufweisen.

10. Bandscheibenprothese gemäß einem der Ansprüche 1 bis 9, wobei die freien Enden an dem schleifenförmigen Bereich angrenzen.

## Revendications

1. Prothèse de disque intervertébral comprenant
un plateau de base (1),
un plateau supérieur (2), un axe central (M) s'étendant à travers le centre du plateau de base et le plateau supérieur,
au moins deux ressorts (4) agencés entre le plateau de base et le plateau supérieur, les ressorts ayant chacun une section (6) en forme de boucle et deux extrémités libres (5a, 5b), où
l'une des extrémités libres (5a) de chaque ressort est reliée au plateau de base et l'autre des extrémités libres est reliée au plateau supérieur, **caractérisé en ce que** la section en forme de boucle est éloignée de l'axe central, et
qu'une pluralité de ressorts (4) en forme de boucle sont prévus dans un agencement circulaire autour de l'axe central (M), où les ressorts sont adjacents l'un à l'autre dans une direction circonférentielle dudit agencement circulaire avec un espace entre les ressorts (4) adjacents.

2. Prothèse de disque intervertébral selon la revendication 1, où le ressort (4) est essentiellement une bande droite.

3. Prothèse de disque intervertébral selon la revendication 1 ou 2, où un dispositif (7) de serrage est prévu lequel serre les sections (5a, 5b) d'extrémités libres au plateau de base (1) et au plateau supérieur (2)

4. Prothèse de disque intervertébral selon une des revendications 1 à 3, où le plateau de base (1) et le plateau supérieur (2) chacune ayant une projection (1b, 2b) coaxiale avec l'axe central, les projections étant en vis-à-vis.

5. Prothèse de disque intervertébral selon la revendication 4, où les sections (5a, 5b) d'extrémités des ressorts sont serrées aux projections (1b, 2b).

6. Prothèse de disque intervertébral selon l'une des revendications 1 à 5, où un élément de serrage (12) est agencé entre le plateau de base et le plateau supérieur, l'élément de serrage étant de préférence un élastomère.

7. Prothèse de disque intervertébral selon l'une des revendications 1 à 6, où les ressorts (4) sont réalisés en un matériau présentant une super-élasticité, de préférence en un alliage à mémoire de forme tel que le nickel-titane.

8. Prothèse de disque intervertébral selon l'une des revendications 1 à 7, où un manchon de protection (13) souple est agencé entre le plateau de base et le plateau supérieur.

9. Prothèse de disque intervertébral selon l'une des revendications 1 à 8, où les côtés du plateau de base et du plateau supérieur qui sont en regard des ressorts (4) comprennent une structure d'engagement (3) pour un engagement avec une extrémité de plaque adjacente d'une vertèbre.

10. Prothèse de disque intervertébral selon l'une des revendications 1 à 9, où les extrémités libres sont adjacentes à la section en forme de boucle.
